# FASCICULE DE BREVET EUROPEEN

(11) **EP 1 525 188 B1**
(45) Date de publication et mention de la délivrance du brevet: **09.07.2008**
(21) Numéro de dépôt: 03753648.9
(22) Date de dépôt: 16.07.2003
(51) Int. Cl.: C07C 323/66, A61K 31/185, A61K 31/255, A61P 9/12

(54) **DERIVES DE 4,4 -DITHIOBIS- (3-AMINOBUTANE-1-SULFONATES) NOUVEAUX ET COMPOSITIONS LES CONTENANT**
4,4' -DITHIOBIS- (3-AMINOBUTANE-1-SULFONATE)-DERIVATE UND SIE ENTHALTENDE ZUSAMMENSETZUNGEN
NOVEL DERIVATIVES OF 4,4'-DITHIOBIS-(3-AMINOBUTANE-1-SULPHONATES) AND COMPOSITIONS CONTAINING SAME

(30) Priorité: 16.07.2002 FR 0208977; 20.03.2003 FR 0303425
(43) Date de publication de la demande: 27.04.2005
(73) Titulaire: INSTITUT NATIONAL DE LA SANTE ET DE LA RECHERCHE MEDICALE (INSERM), 75654 Paris Cédex 13 (FR)
(72) Inventeur: FOURNIE-ZALUSKI, Marie-Claude, F-75011 Paris (FR); LLORENS-CORTES, Catherine, F-91440 Bures sur Yvette (FR); ROQUES, Bernard, P., F-75014 Paris (FR); CORVOL, Pierre, F-75007 Paris (FR)
(74) Mandataire: Santarelli
(86) Numéro de dépôt international: PCT/FR2003/002242
(87) Numéro de publication internationale: WO 2004/007441

(56) Documents cités:
- WO-A-99/36066
- E.N. CHAUVEL, ET AL.: "Investigation of the active site of aminopeptidase a using a seriesof new thiol-containing inhibitors" JOURNAL OF MEDICINAL CHEMISTRY, vol. 37, no. 9, 29 avril 1994 (1994-04-29), pages 1339-1346, XP002237215 American Chemical Society, Washington, DC, US ISSN: 0022-2623
- E.N. CHAUVEL, ET AL.: "Differential inhibition of aminopeptidase A and aminopeptidase N by new beta-amino thiols" JOURNAL OF MEDICINAL CHEMISTRY, vol. 37, no. 18, 2 septembre 1994 (1994-09-02), pages 2950-2957, XP002204204 American Chemical Society, Washington, DC, US ISSN: 0022-2623

## Description

La présente invention a pour objet des composés nouveaux, des procédés pour la préparation de ces composés, des formulations pharmaceutiques comprenant ces composés, et l'utilisation de ces composés en thérapeutique. La présente invention concerne en particulier des composés qui sont utiles dans le traitement et la prévention de l'hypertension artérielle primaire et secondaire, d'un ictus, de l'ischémie myocardique, de l'insuffisance cardiaque et de l'insuffisance rénale, de l'infarctus du myocarde, d'une maladie vasculaire périphérique, de la protéinurie diabétique, du syndrome X et du glaucome.

L'hypertension artérielle est une affection dont les causes restent généralement inconnues. Des facteurs extrinsèques qui peuvent participer comprennent l'obésité, un mode de vie sédentaire, l'absorption excessive d'alcool ou de sel et le stress. Des facteurs intrinsèques suggérés en tant que facteurs jouant un rôle comprennent la rétention de fluide, l'activité du système nerveux sympathique et la constriction des vaisseaux sanguins. L'hypertension artérielle peut contribuer directement ou indirectement à des maladies du coeur, du système vasculaire périphérique et cérébral, du cerveau, de l'oeil et du rein.

Le traitement de l'hypertension artérielle comprend l'utilisation d'agents diurétiques, d'agents de blocage adrénergique, d'inhibiteurs de l'enzyme de conversion d'angiotensine, d'antagonistes des récepteurs d'angiotensine, d'antagonistes du calcium et de vasodilatateurs directs. Il est souhaitable d'identifier des composés supplémentaires pour le traitement de l'hypertension artérielle.

Le document WO 99/36066 décrit le sel de sodium de l'acides (S)-3-amino-4-mercaptobutanesulfonique comme inhibiteur sélectif de l'aminopeptidase A et utile pour diminuer la pression artérielle.

Les présents inventeurs ont identifié des composés nouveaux qui sont efficaces dans la réduction de l'hypertension artérielle et qui, ainsi, sont utiles dans le traitement de l'hypertension artérielle et des maladies auxquelles elle contribue indirectement et directement.

En conséquence, la présente invention comprend les composés suivants :
4,4'-dithiobis-(sel de sodium d'acide 3-aminobutane-1-sulfonique) ;
4,4'-dithiobis-(3-aminobutane-1-sulfonate de 2,2-diméthylpropyle).

Dans un autre aspect, la présente invention décrit une méthode pour la prévention ou le traitement de l'hypertension artérielle et de maladies indirectement et directement apparentées, comprenant l'administration d'une quantité thérapeutiquement efficace d'un composé de la présente invention. Dans un autre aspect, la présente invention propose des compositions pharmaceutiques comprenant un ou plusieurs composés de la présente invention, de préférence en association avec un diluant ou support pharmaceutiquement acceptable.

Dans un autre aspect, la présente invention propose un ou plusieurs composés de la présente invention à des fins d'utilisation en thérapeutique, et en particulier en médecine humaine.

Dans un autre aspect, la présente invention propose l'utilisation d'un ou plusieurs composés de la présente invention pour la production d'un médicament destiné au traitement de l'hypertension artérielle et de maladies indirectement et directement apparentées.

Dans un autre aspect, la présente invention propose une méthode de traitement d'un patient souffrant d'hypertension artérielle et de maladies indirectement et directement apparentées, comprenant l'administration d'une quantité thérapeutiquement efficace d'un ou plusieurs composés de la présente invention.

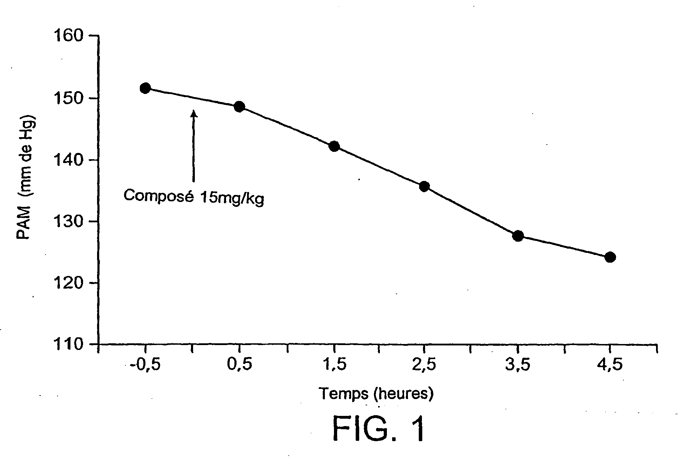

La figure 1 démontre l'effet du composé de l'Exemple 1 sur la pression sanguine chez des rats hypertensifs.

La présente invention propose des méthodes pour la prévention ou le traitement de l'hypertension artérielle et de maladies auxquelles l'hypertension artérielle contribue directement ou indirectement. Ces maladies comprennent des maladies du coeur, du système vasculaire périphérique et cérébral, du cerveau, de l'oeil et du rein. En particulier, les maladies comprennent l'hypertension artérielle primaire et secondaire, un ictus, l'ischémie myocardique, l'insuffisance cardiaque et l'insuffisance rénale, l'infarctus du myocarde, une maladie vasculaire périphérique, la protéinurie diabétique, le syndrome X et le glaucome.

Telle qu'elle est utilisée dans le présent mémoire, l'expression "composé de la présente invention" désigne un composé décrit ci-dessus ou un de ses sels ou produit de solvatation pharmaceutiquement acceptable.

L'homme de l'art reconnaîtra que des stéréocentres existent dans les composés de la présente invention. En conséquence, la présente invention comprend tous les stéréo-isomères et isomères géométriques possibles des composés selon l'invention et comprend non seulement des composés racémiques mais également les isomères optiquement actifs. Lorsqu'un composé selon l'invention est désiré sous forme d'un énantiomère unique, il peut être obtenu par résolution du produit final ou par synthèse stéréospécifique à partir de la matière de départ isomériquement pure ou bien de n'importe quel intermédiaire convenable. La résolution du produit final, d'un intermédiaire ou d'une matière de départ peut être effectuée par n'importe quel procédé convenable connu dans ce domaine. Voir, par exemple, Stereochemistry of Carbon Compounds par E. L. Eliel (Mcgraw Hill, 1962) et Tables of Resolving Agents par S. H. Wilen. En outre, dans les cas où des formes tautomères des composés selon l'invention sont possibles, la présente invention est destinée à comprendre toutes les formes tautomères des composés.

Le spécialiste de la chimie organique notera que de nombreux composés organiques peuvent former des complexes avec des solvants dans lesquels ils ont été amenés à réagir ou à partir desquels ils sont précipités ou cristallisés. Ces complexes sont connus sous le nom de "produits de solvatation". Par exemple, un complexe avec l'eau est connu sous le nom de "hydrate". Les produits de solvatation du composé selon l'invention entrent dans le cadre de la présente invention.

Le spécialiste de la chimie organique notera également que de nombreux composés organiques peuvent exister sous plus d'une forme cristalline. Par exemple, la forme cristalline peut varier d'un produit de solvatation à l'autre. Ainsi, toutes les formes cristallines des composés de la présente invention ou de leurs produits de solvatation pharmaceutiquement acceptables sont incluses dans le cadre de la présente invention.

L'homme de l'art notera également que, en plus de l'utilisation sous forme du composé mère, les composés de la présente invention peuvent être également utilisés sous forme de leur sels ou produits de solvatation pharmaceutiquement acceptables. Les sels pharmaceutiquement acceptables des composés de la présente invention comprennent des sels classiques formés à partir d'acides ou de bases inorganiques ou organiques pharmaceutiquement acceptables ainsi que des sels d'ammonium quaternaire. Des exemples plus spécifiques de sels d'acides convenables comprennent les sels formés avec les acides chlorhydrique, bromhydrique, sulfurique, phosphorique, nitrique, perchlorique, fumarique, acétique, propionique, succinique, glycolique, formique, lactique, maléique, tartrique, citrique, palmoïque, malonique, hydroxymaléique, phénylacétique, glutamique, benzoïque, salicylique, fumarique, toluènesulfonique, méthanesulfonique, naphtalène-2-sulfonique, benzènesulfonique, hydroxy-naphtoïque, iodhydrique, malique, stéroïque, tannique, etc. D'autres acides tels que l'acide oxalique, bien que n'étant pas en eux-mêmes pharmaceutiquement acceptables, peuvent être utiles dans la préparation de sels utiles comme intermédiaires dans l'obtention des composés de la présente invention et de leurs sels pharmaceutiquement acceptables. Des exemples plus spécifiques de sels basiques convenables comprennent les sels de sodium, de lithium, de potassium, de magnésium, d'aluminium, de calcium, de zinc, de N,N'-dibenzyléthylène-diamine, de chloroprocaïne, de choline, de diéthanolamine, d'éthylènediamine, de N-méthylglucamine et de procaïne. Des références ci-après à un composé conforme à la présente invention concernent à la fois les composés de formule (I) et leurs sels et produits de solvatation pharmaceutiquement acceptables.

Par exemple, les formes de sels préférées comprennent :
le bis-chlorhydrate de 4,4'-dithiobis(3-aminobutane-1-sulfonate de sodium) ;
le bis-trifluoracétate de 4,4'-dithiobis(3-aminobutane-1-sulfonate de 2,2-diméthylpropyle).

Les composés de la présente invention et leurs dérivés pharmaceutiquement acceptables sont administrés convenablement sous forme de compositions pharmaceutiques. Ces compositions peuvent être présentées convenablement à des fins d'utilisation de manière classique en mélange avec un ou plusieurs supports ou excipients physiologiquement acceptables. Le ou les supports doivent être "acceptables" en ce sens qu'ils doivent être compatibles avec les autres ingrédients de la formulation et ils ne doivent pas être néfastes pour le sujet les recevant.

Bien qu'il soit possible d'administrer thérapeutiquement les composés de la présente invention sous forme de la substance chimique brute, il est préférable de présenter l'ingrédient actif sous forme d'une formulation pharmaceutique.

En conséquence, la présente invention propose en outre une formulation pharmaceutique comprenant un composé de la présente invention ou un de ses sels ou produit de solvatation pharmaceutiquement acceptable en association avec un ou plusieurs supports pharmaceutiquement acceptables et, facultativement, d'autres ingrédients thérapeutiques et/ou prophylactiques.

Les formulations comprennent celles convenables pour l'administration orale, parentérale (y compris sous-cutanée, par exemple par injection ou au moyen d'un comprimé à dépôt, intradermique, intrathécal, intramusculaire, par exemple par dépôt, et intraveineuse), rectale et topique (y compris dermique, buccale et sublinguale) ou sous une forme convenable pour l'administration par inhalation ou insufflation, bien que la voie convenant le mieux puisse dépendre, par exemple, de l'état et de l'affection du receveur. Les formulations peuvent être présentées convenablement sous une forme posologique unitaire et peuvent être préparées par n'importe lequel des procédés bien connus dans le domaine de la pharmacie. Tous les procédés comprennent l'étape consistant à mettre en association les composés ("ingrédients actifs") avec le support qui comprend un ou plusieurs ingrédients accessoires. En général, les formulations sont préparées en mettant uniformément et intimement en association l'ingrédient actif avec des véhicules liquides ou des supports solides finement divisés ou bien avec ces deux types de supports et ensuite, si nécessaire, en façonnant le produit en la formulation désirée.

Les formulations convenables pour l'administration orale peuvent être présentées sous forme d'unités discrètes telles que des capsules, cachets ou comprimés (par exemple des comprimés à mâcher, en particulier pour une administration pédiatrique), chacun contenant une quantité prédéterminée de l'ingrédient actif ; sous forme d'une poudre ou de granules ; sous forme d'une solution ou d'une suspension dans un liquide aqueux ou un liquide non aqueux ; ou sous forme d'une émulsion liquide huile-dans-eau ou d'une émulsion liquide eau-dans-huile. L'ingrédient actif peut également être présenté sous forme d'un bol, d'un électuaire ou d'une pâte.

Un comprimé peut être préparé par compression ou moulage, facultativement avec un ou plusieurs ingrédients accessoires. Des comprimés produits par compression peuvent être préparés en comprimant dans une machine convenable l'ingrédient actif sous une forme à écoulement libre telle qu'une poudre ou des granules, facultativement en mélange avec d'autres excipients classiques tels que des liants (par exemple un sirop, la gomme arabique, la gélatine, le sorbitol, la gomme adragante, un mucilage d'amidon, la polyvinylpyrrolidone ou l'hydroxyméthylcellulose), des charges (par exemple le lactose, le saccharose, la cellulose microcristalline, l'amidon de maïs, le phosphate de calcium ou le sorbitol), des lubrifiants (par exemple le stéarate de magnésium, l'acide stéarique, le talc, le polyéthylèneglycol ou la silice), des agents de délitement (par exemple la fécule de pomme de terre ou le glycolate d'amidon sodique) ou des agents mouillants tels que le laurylsulfate de sodium. Des comprimés moulés peuvent être préparés en moulant dans une machine convenable un mélange du composé pulvérisé humidifié avec un diluant liquide inerte. Les comprimés peuvent être facultativement enrobés ou entaillés et peuvent être formulés de manière à provoquer la libération lente ou contrôlée de l'ingrédient actif qui s'y trouve. Les comprimés peuvent être enrobés par des procédés bien connus dans ce domaine.

En variante, les composés de la présente invention peuvent être incorporés à des préparations liquides orales telles que des suspensions, solutions ou émulsions aqueuses ou huileuses, des sirops ou élixirs, par exemple. En outre, des formulations contenant ces composés peuvent être présentées à l'état de produits secs destinés à une reconstitution avec de l'eau ou un autre véhicule convenable avant utilisation. Ces préparations liquides peuvent contenir des additifs classiques tels que des agents de mise en suspension, par exemple le sirop de sorbitol, la méthylcellulose, le glucose/sirop de sucre, la gélatine, l'hydroxyéthylcellulose, la carboxyméthylcellulose, un gel de stéarate d'aluminium ou des matières grasses comestibles hydrogénées ; des agents émulsionnants tels que la lécithine, le mono-oléate de sorbitanne ou la gomme arabique ; des véhicules non aqueux (qui peuvent comprendre des huiles comestibles) tels que l'huile d'amande, l'huile de coprah fractionnée, des esters huileux, le propylèneglycol ou l'alcool éthylique ; et des conservateurs tels que le p-hydroxybenzoate de méthyle ou de propyle ou l'acide sorbique. Ces préparations peuvent également être formulées à l'état de suppositoires, contenant par exemple des excipients classiques pour suppositoires tels que le beurre de cacao ou d'autres glycérides.

Les formulations pour l'administration parentérale comprennent des solutions injectables stériles aqueuses et non aqueuses qui peuvent contenir des antioxydants, des tampons, des agents bactériostatiques et des solutés qui rendent la formulation isotonique avec le sang du receveur choisi ; et des suspensions aqueuses et non aqueuses stériles qui peuvent comprendre des agents de mise en suspension et des agents épaississants. Les formulations peuvent être présentées dans des récipients à dose unique ou doses multiples, par exemple des ampoules et flacons clos hermétiquement, et peuvent être stockées à l'état séché par congélation (lyophilisé) nécessitant seulement l'addition d'un véhicule liquide stérile, par exemple de l'eau pour préparations injectables, immédiatement avant utilisation. Des solutions et suspensions injectables extemporanées peuvent être préparées à partir de poudres, granules et comprimés stériles du type décrit précédemment.

Les formulations pour l'administration rectale peuvent être présentées à l'état de suppositoires avec les supports usuels tels que le beurre de cacao, une graisse dure ou le polyéthylèneglycol.

Les formulations pour l'administration topique dans la cavité buccale, par exemple pour l'administration buccale ou sublinguale, comprennent des tablettes comprenant l'ingrédient actif dans un excipient aromatisé tel que la saccharose et la gomme arabique ou la gomme adragante, et des pastilles comprenant l'ingrédient actif dans un excipient tel que la gélatine et le glycérol ou le saccharose et la gomme arabique.

Pour l'administration topique à l'épiderme, les composés peuvent être formulés à l'état de crèmes, de gels, de pommades ou de lotions ou sous forme d'un timbre transdermique.

Les composés peuvent également être formulés à l'état de préparations pour dépôt. Ces formulations à longue durée d'action peuvent être administrées par implantation (par exemple par voie sous-cutanée ou intramusculaire) ou bien par injection intramusculaire. Ainsi, par exemple, les composés peuvent être formulés avec des matières polymères ou hydrophobes convenables (par exemple sous forme d'une émulsion dans une huile acceptable) ou des résines échangeuses d'ions, ou sous forme de dérivés très faiblement solubles, par exemple sous forme d'un sel très faiblement soluble.

Pour l'administration intranasale, les composés de la présente invention peuvent être utilisés, par exemple, sous forme d'un liquide d'atomisation, d'une poudre ou de gouttes.

Pour l'administration par inhalation, les composés conformes à la présente invention sont délivrés convenablement sous forme d'un aérosol émis par pulvérisation par un récipient sous pression ou un nébuliseur, au moyen d'un agent propulseur convenable, par exemple le 1,1,1,2-trifluoréthane (HFA 134A) et le 1,1,1,2,3,3,3-heptafluoropropane (HFA 227), le dioxyde de carbone ou un autre gaz convenable. Dans le cas d'un aérosol sous pression, la dose exacte peut être déterminée en installant une valve destinée à délivrer une quantité mesurée. Les capsules et cartouches constituées, par exemple, de gélatine, destinées à être utilisées dans un inhalateur ou insufflateur peuvent être formulées de manière à contenir un mélange de poudres constitué d'un composé de la présente invention et d'un excipient en poudre convenable tel que le lactose ou l'amidon.

En plus des ingrédients mentionnés particulièrement ci-dessus, les formulations peuvent comprendre d'autres agents classiques dans ce domaine en rapport avec le type de formulation en question ; par exemple, les formulations convenables pour l'administration orale peuvent comprendre des agents aromatisants.

L'homme de l'art notera qu'une référence dans le présent mémoire à un traitement s'étend à la prophylaxie ainsi qu'au traitement de maladies ou symptômes établis. En outre, on notera que la quantité d'un composé de la présente invention requise pour l'utilisation dans un traitement varie en fonction de la nature de l'affection traitée et de l'âge et de l'état du patient et sera finalement laissée à la discrétion du médecin traitant ou vétérinaire. Cependant, en général, les doses utilisées pour le traitement d'un patient humain adulte sont comprises habituellement dans l'intervalle de 0,02 à 5000 mg par jour, de préférence de 1 à 1500 mg par jour. La dose désirée peut être présentée convenablement en une dose unique ou de manière fractionnée en plusieurs doses administrées à des intervalles appropriés, par exemple sous forme de deux, trois, quatre ou plus de quatre doses secondaires par jour. Les formulations conformes à la présente invention peuvent contenir 0,1 à 99 % de l'ingrédient actif, convenablement 30 à 95 % pour les comprimés et capsules et 3 à 50 % pour les préparations liquides.

Le composé de la présente invention destiné à être utilisé dans la présente invention peut être utilisé en association avec un ou plusieurs autres agents thérapeutiques, par exemple des antagonistes des récepteurs bêta-adrénergiques, des agents de blocage du canal calcium, des diurétiques du type thiazide, des antagonistes des récepteurs d'angiotensine et des inhibiteurs de l'enzyme de conversion d'angiotensine. Ainsi, la présente invention propose dans un aspect supplémentaire l'utilisation d'une association comprenant un composé de formule (I) et d'un agent thérapeutique supplémentaire dans le traitement de l'hypertension artérielle.

Lorsque les composés de la présente invention sont utilisés en association avec d'autres agents thérapeutiques, les composés peuvent être administrés successivement ou simultanément par n'importe quelle voie convenable.

Les associations mentionnées ci-dessus peuvent être présentées convenablement à des fins d'utilisation sous forme d'une formulation pharmaceutique et, ainsi, des formulations pharmaceutiques comprenant une association répondant à la définition précitée conjointement de manière optimale avec un support ou excipient pharmaceutiquement acceptable constituent un aspect supplémentaire de la présente invention. Les différents constituants de ces associations peuvent être administrés successivement ou simultanément dans des formulations pharmaceutiques séparées ou combinées.

Lorsqu'ils sont combinés dans la même formulation, on notera que les deux composés doivent être stables et compatibles l'un avec l'autre et les autres constituants de la formulation et peuvent être formulés pour l'administration. Lorsqu'ils sont formulés séparément, ils peuvent être fournis dans n'importe quelle formulation convenable, commodément d'une manière connue pour de tels composés dans ce domaine.

Lorsqu'un composé de la présente invention est utilisé en association avec un second agent thérapeutique actif contre la même maladie, la dose de chaque composé peut différer de celle administrée lorsque le composé est utilisé seul. Les doses appropriées seront aisément déterminées par l'homme de l'art.

Les composés de la présente invention peuvent être préparés au moyen des exemples suivants qui ne doivent pas être considérés comme constituant une limitation de la présente invention.

### Exemple 1 bis-chlorhydrate de 4,4'-dithiobis-(3-aminobutane-1-sulfonate de sodium)

### Etape 1 : Synthèse du chlorhydrate de 2-amino-4-chloro-1-éthoxycarbonylpropane

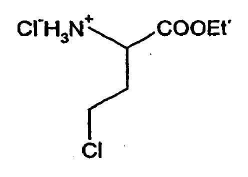

Une solution de 20 g de L-homosérine dans 50 ml d'éthanol absolu a été refroidie à 0°C et 121 ml (10 équivalents) de SOCl₂ ont été ajoutés goutte à goutte. Le mélange a été réchauffé à température ambiante et ensuite chauffé au reflux pendant 8 heures. La solution a été évaporée sous vide et le résidu a été traité avec Et₂O. Le précipité a été filtré et lavé trois fois avec Et₂O. Substance solide blanche : 31,2 g (92 %). Rf (CH₂Cl_{2/}MeOH/ AcOH : 7/3/0,5) 0,59.

### Etape 2 : Synthèse du 2-tertiobutoxycarbonylamino-4-chloro-butanoate d'éthyle

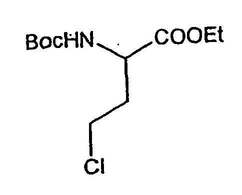

Le composé précédent (31,2 g) dissous dans 80 ml de DMF, a été refroidi à - 10°C, puis une solution de (Boc)₂O (37,1 g) dans 80 ml de DMF et 23,8 ml de Et₃N a été ajoutée. Le mélange a été agité à température ambiante pendant une nuit. La solution a été évaporée sous vide et le résidu a été partagé entre H₂O et Et₂O. La phase organique a été lavée, déshydratée sur Na₂SO₄, filtrée et évaporée sous vide. Substance solide beige : 40,7 g (99 %). Rf (EtOAc/n-Hex : 3/1) 0,66.

### Etape 3 : Synthèse du 3-tertiobutoxycarbonylamino-3-éthoxycarbonylpropane-1-sulfonate de sodium

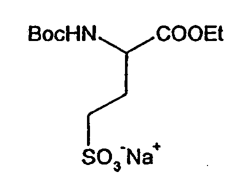

Le composé précédent (10,8 g) a été dissous dans un mélange de 150 ml de dioxanne/150 ml de H₂O et 6,1 g de NaI et 25,6 g de Na₂SO₃ ont été ajoutés. Le mélange a été chauffé au reflux pendant 15 heures, puis évaporé sous vide. Le résidu a été dissous dans EtOH (250 ml). Le précipité a été éliminé et le filtrat a été évaporé sous vide. Une poudre blanche a été obtenue ; 12 g (89 %). Rf (CH₂Cl₂/MeOH : 8/2) 0,18.

### Etape 4 : Synthèse du 3-tertiobutoxycarbonylamino-4-hydroxybutane-1-sulfonate de sodium

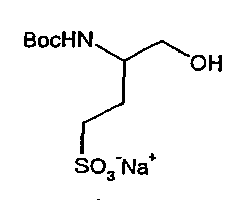

L'ester précédent (10 g) a été dissous dans 125 ml de EtOH absolu et 125 ml de THF anhydre, puis 5,1 g de LiCl anhydre et 4,9 g de NaBH₄ ont été ajoutés. Le mélange a été agité pendant 17 heures à température ambiante. De l'acide acétique (60 ml) a été ajouté à 0°C et le mélange a été évaporé sous vide. Le produit brut a été purifié par chromatographie sur du gel de silice en utilisant comme éluant un mélange EtOAc/MeOH dans le rapport 8/2. Substance solide blanche : 7,16 g (82 %). Rf (EtOAc/MeOH : 7 / 3) 0,32.

### Etape 5 : Synthèse du 4-acétylsulfanyl-3-tertiobutoxycarbonylaminobutane-1-sulfonate de sodium

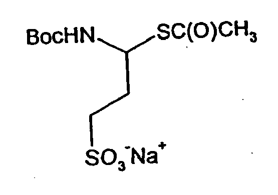

Une solution de 13 g de triphénylphosphine dans du THF anhydre (170 ml) a été refroidie à 0°C et 10 ml d'azodicarboxylate de diisopropyle ont été ajoutés. La solution a été agitée pendant 45 minutes à la même température. Une solution de l'alcool précédent (7 g) dans du THF (125 ml) + du DMF (40 ml) a été ajoutée et a été suivie, 15 minutes plus tard, par 4 ml de CH₃COSH et le mélange a été agité pendant une nuit à température ambiante. Après évaporation sous vide, le résidu a été dissous dans EtOAc et lavé avec une solution de NaHCO₃ (à 10 %), H₂O et de la saumure et a été déshydraté sur Na₂SO₄.

Après évaporation, un mélange n-hexane/EtOAc a été ajouté et le précipité a été éliminé. Le filtrat a été évaporé et le résidu a été purifié par chromatographie sur du gel de silice en utilisant comme éluant un mélange n-Hex/EtOAc dans le rapport 4 : 1. Produit huileux : 8,4 g (80 %). Rf (CH₂Cl_{2/}MeOH : 8/2) 0,20.

### Etape 6 : Synthèse du bis-chlorhydrate de 4,4'-dithiobis-(3-aminobutane-1-sulfonate de sodium).

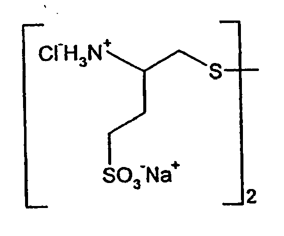

350 mg du composé précédent ont été chauffés au reflux avec 15 ml de HCl 6 N pendant 3 heures. La solution a été évaporée sous vide et le résidu a été dissous dans un mélange EtOH/H₂O dans le rapport 1/4 et a été traité avec une solution d'iode jusqu'à observation d'une couleur jaune persistante. La solution a été évaporée et le composé final a été précipité avec Et₂O. Substance solide blanche hautement hygroscopique : 200 mg (80 %).

En variante, le composé mère peut être préparé à partir du composé à fonction thiole libre de la manière suivante :

7,0 g de EC33 sont dissous dans 100 ml de méthanol sous agitation. Une solution de 7,32 g d'iode dans 100 ml de méthanol est ajoutée goutte à goutte jusqu'à cessation de la décoloration. Le précipité résultant est filtré et lavé avec des volumes de 20 ml de méthanol jusqu'à ce que les liquides de lavage soient incolores. Le précipité est lavé à l'éther et séché sous pression réduite, ce qui donne 4,1 g d'une substance solide blanche.
[α]_{D}²⁰ = + 194,5 eau, c = 1,33 ;
Calculé % C 26,07, H 5,47, N 7,60, O 26,05, S 34,81
Trouvé % C 25,61, H 5,60, N 7,39, O 25,99, S 33,50 ;
RMN (D₂O, 400 MHz) : δ 2,1 (m, 2H, CH₂β) ; 2,85 (dd, 1H, CH₂γ) ; 2,95 (t, 2H, CH₂β') ; 3,10 (dd, 1H, CH₂γ) ; 3,70 (m, 1H, CH α).
Rf = 0,26 dans un mélange isopropanol/eau/acide acétique dans le rapport 8/2/1, volume/volume/volume.

### Exemple 2 Bis-trifluoracétate de 4,4'-dithiobis-(3-aminobutane-1-sulfonate de 2,2-diméthylpropyle)

### Etape 1 : Benzyloxycarbonyl-L-homocystine

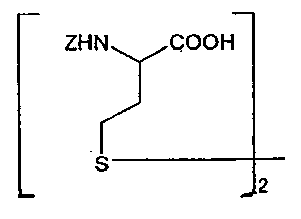

De la L-homocystine (5 g) a été dissoute dans un mélange (80 ml) de dioxanne/H₂O. A 0°C et sous agitation, une quantité de 1,52 g (2,1 équivalents) de NaOH et une solution de 7,8 g (2,4 équivalents) de chloroformiate de benzyle dans 40 ml de dioxanne ont été ajoutées. Le pH a été maintenu à 9 par addition d'une solution de NaOH 1 M. Après agitation pendant 2,30 heures à température ambiante, 100 ml de H₂O ont été ajoutés et le précipité blanc a été soumis à une extraction avec Et₂O (2 x 50 ml). La phase aqueuse a été acidifiée à pH 1 et le précipité a été soumis à une extraction avec EtOAc (4 x 80 ml). La phase organique a été lavée, déshydratée sur Na₂SO₄, filtrée et évaporée sous vide. Substance solide blanche : 10,2 g (100 %).

### Etape 2 : Benzyloxycarbonyl-L-homocystinate d'éthyle

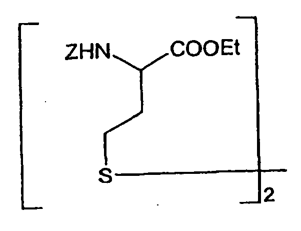

De la Z-L-homocystine (10 g), a été dissoute dans 150 ml de EtOH absolu. Une solution de 1 ml de SOCl₂ dans CH₂Cl₂ (17 ml) a été ajoutée à 0°C et le mélange a été chauffé au reflux pendant 4 heures. Le mélange a été évaporé sous vide et le résidu a été dissous dans CH₂Cl₂. La phase organique a été lavée, déshydratée sur Na₂SO₄, filtrée et évaporée sous vide. Pâte jaune : 9 g (80 %). Rf (EtOAc/cHex : 1/1) 0,59.

### Etape 3 : 2-benzyloxycarbonylamino-4-(2,2-diméthylpropyl)-1-sulfonylbutanoate d'éthyle

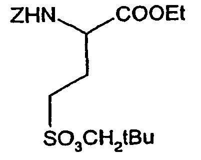

Le composé précédent (9 g) a été dissous dans un mélange de CCl₄/EtOH et du Cl₂ gazeux a été passé par barbotage à travers le mélange pendant 45 minutes. Après évaporation sous vide, on a obtenu une pâte de couleur jaune que l'on a dissous dans 200 ml de CH₂Cl₂. Puis, 3,48 g d'alcool néopentylique et 5,85 ml de Et₃N ont été ajoutés. Le mélange a été agité pendant une nuit, évaporé sous vide et purifié par chromatographie sur du gel de silice, en utilisant comme éluant un mélange EtOAc/cHex dans le rapport 1/4. 11,2 g d'une substance solide blanche ont été obtenus (90 %). Rf (EtOAc/cHex : 1/4) 0,16.

### Etape 4 : 2-tertiobutoxycarbonylamino-4-(2,2-diméthylpropyl)-1-sulfonylbutanoate d'éthyle

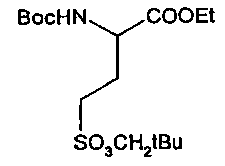

Le composé précédent (5,2 g) a été dissous dans 30 ml de EtOAc et une solution de 4,07 g de Boc₂O dans 30 ml de EtOAc et 400 mg d'un catalyseur à 10 % de Pd/C ont été ajoutés. Le mélange a été agité sous une pression de H₂ de 250 kPa à 40°C pendant 48 heures. Le mélange a été filtré sur de la Celite et la phase organique a été évaporée sous vide (100 %). Rf (EtOAc/cHex : 1/4) 0,79.

### Etape 5 : 3-tertiobutoxycarbonylamino-4-hydroxybutane-1-sulfonate de (2,2-diméthylpropyle)

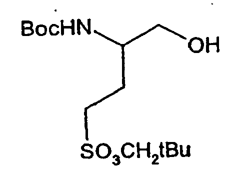

Le composé précédent (2,44 g) a été dissous dans 120 ml d'un mélange THF/EtOH dans le rapport 50/50. La solution a été refroidie à - 10°C sous atmosphère inerte et 1,09 g (4 équivalents) de LiCl et 0,97 g (4 équivalents) de NaBH₄ ont été ajoutés. Après un temps de 15 minutes à - 10°C, le mélange a été agité à température ambiante pendant 60 heures. Puis 20 ml de AcOH ont été ajoutés et le mélange a été évaporé sous vide. Le résidu a été dissous dans 400 ml de EtOAc, lavé avec de l'eau et de la saumure et déshydraté sur Na₂SO₄. Le produit brut a été purifié par chromatographie sur du gel de silice en utilisant comme éluant un mélange EtOAc/MeOH/cHex dans le rapport 1/1/4 (Rf 0,20) : 2,1 g (99 %).

### Etape 6 : 3-tertiobutyloxycarbonylamino-4-acétylsulfanylbutane-1-sulfonate de (2,2-diméthylpropyle)

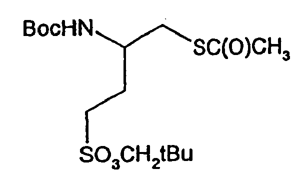

Le composé précédent (0,965 g) dans 10 ml de CHCl₃ a été refroidi à - 10°C et 1,07 ml de Et₃N et 0,44 ml de CH₃SO₂Cl dans 4 ml de CHCl₃ ont été ajoutés successivement. Le mélange a été agité à température ambiante pendant 1,5 heure. Puis 40 ml de CHCl₃ ont été ajoutés et la phase organique a été lavée à 0°C avec une solution à 10 % de NaHCO₃, H₂O, HCl 1 N, H₂O et de la saumure et a été déshydratée sur Na₂SO₄. Après filtration et évaporation, le produit brut (Rf (EtOAc/AcOH/cHex : 1/1/4) = 0,41) a été dissous dans 15 ml de DMF et, à - 10°C, une quantité de 0,65 g de CH₃COSK a été ajoutée. Le mélange a été agité pendant deux jours à température ambiante. Le solvant a été évaporé sous vide et un résidu orange a été obtenu.

Chromatographie sur du gel de silice : éluant EtOAc/cHex dans le rapport 1/4 (Rf = 0,15) ; substance solide blanche : 0,64 g (57 %).

### Etape 7 : 4,4'-dithiobis-(3-tertiobutyloxycarbonylaminobutane-1-sulfonate) de 2,2-diméthylpropyle

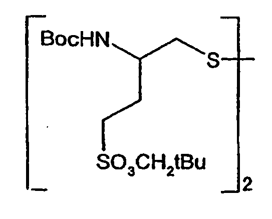

Le composé précédent (0,25 g) a été dissous dans un mélange EtOH/THF dans le rapport 2/1. Puis 60 mg de NaOH dissous dans 1 ml de H₂O ont été ajoutés. Le mélange a été agité tout en y faisant barboter du O₂ pendant 12 heures. Après évaporation sous vide, le résidu a été dissous dans un mélange de 40 ml de H₂O/40 ml de EtOAc et a été acidifié à pH 1. La phase organique a été isolée, lavée, déshydratée sur Na₂SO₄, filtrée et évaporée sous vide. Substance solide blanche : 0,178 g (80 %). Rf (EtOAc/MeOH/cHex : 1/1/4) 0,28.

### Etape 8 : Bis-trifluoracétate de 4,4'-dithiobis-(3-aminobutane-1-sulfonate) de 2,2-diméthylpropyle

Le disulfure précédent (0,17 g) a été dissous dans 6 ml de CH₂Cl₂ et 6 ml de CF₃CO₂H ont été ajoutés. Le mélange a été agité à température ambiante pendant 2 heures et évaporé sous vide. Le résidu a été lavé avec Et₂O. Substance solide blanche : 0,17 g (100 %). Rf (CH₂Cl₂/MeOH : 7/3) 0,47.
[α]_{D}¹⁹ = + 24,8 c = 0,995 dans EtOH 95 %
¹H-RMN (DMSO) : δ 0,90 (s, 9H, tBu) ; 2,1 (m, 2H, CH₂β) ; 2,70-2,75 (m, 1H, CH₂β') ; 2,80-2,90 (dd, 1H, CH₂β') ; 3,00-3,10 (dd, 1H, CH₂α) ; 3,50 (m, 3H, CH α et CH₂γ) ; 3,90 (s, 2H, CH₂γ'), 8,1 (s, 2H, NH₃⁺)

Ce composé peut être converti en la molécule mère ou d'autres formes de sels convenables par des procédés connus dans ce domaine. Par exemple, pour la conversion en la molécule mère, 40 mg de RB 151 sont dissous dans 2 ml d'eau. 5 ml d'éther sont ajoutés, puis un volume de 0,12 ml d'une solution aqueuse hydroxyde de sodium (1M) est ajouté goutte à goutte. La phase aqueuse devient laiteuse et ensuite se clarifie rapidement. Le mélange est agité pendant 30 minutes et la phase organique est séparée. La phase aqueuse est lavée trois fois avec 5 ml d'éther. Les phases organiques réunies sont déshydratées sur du sulfate de sodium, puis concentrées sous vide, ce qui donne une poudre amorphe en un rendement dé 98 %.
[α]_{D}¹⁹ = + 42,3 c = 0,992 dans EtOH 95 %
¹H-RMN (DMSO) : δ 0,90 (s, 9H, tBu) ; 1,65-1,75 (m, 1H, CH₂β) ; 1,90-2,00 (m, 1H, CH₂β) ; 2,70-2,75 (m, 1H, CH₂β') ; 2,80-2,90 (m, 1H, CH₂β') ; 3,00-3,10 (m, 1H, CH₂α) ; 3,35-3,50 (m, 2H, CH₂γ), 3,80 (s, 2H, CH₂γ')

### EXEMPLE D'ACTIVITE BIOLOGIQUE

### Effet sur la pression sanguine chez le rat

Des rats rendus hypertensifs avec un sel d'acétate de désoxycorticostérone (DOCA) ont été obtenus d'après Pham, I. et al. (1993) J. Pharmacol. Exp. Ther. 265, 1339-1347, avec les modifications suivantes : sous anesthésie au pentobarbital, une néphrectomie unilatérale a été réalisée chez des rats Wistar Kyoto mâles (300 g) et une pastille de 50 g de DOCA a été implantée par voie sous-cutanée. Après l'intervention chirurgicale, les rats ont été nourris avec un aliment classique pour rats et l'eau de boisson a été additionnée de 0,9 % de NaCl et 0,2 % de KCl. Une hypertension est apparue 3 semaines après l'intervention chirurgicale.

Pour l'enregistrement de la pression sanguine artérielle, les rats ayant reçu le sel de DOCA ont été anesthésiés avec du pentobarbital sodique (50 mg/kg par voie i.p., Sentravet Laboratory, Plancoët, France), un cathéter d'artère fémorale (PE₅₀) rempli de sérum physiologique héparinisé (250 U/ml) a été inséré, puis a été passée sous la peau et sorti au niveau de la nuque. Un ressort métallique flexible a été fixé au crâne et au cou du rat et connecté à des rotules à deux canaux montés directement au-dessus de la cage. Cette configuration a permis le mouvement libre du rat à l'intérieur de la cage. Puis chaque rat a reçu une injection intramusculaire de 0,1 ml de pénicilline-streptomycine (50 000 UI/ml, Boehringer Mannheim, GmbH - Allemagne) et a été abandonné pour son rétablissement pendant au moins 24 heures avant l'expérience. La pression sanguine artérielle moyenne a été enregistrée de manière continue pendant toute cette expérience en utilisant un transducteur de pression COBE CDX III (Phymep, Paris, France) connecté au système MacLab (Phymep, Paris, France) constitué d'une unité technologique MacLab et d'un logiciel Chart fonctionnant sur un ordinateur MacIntosh.

Le composé de l'Exemple 1 a été administré aux rats par gavage oral dans de l'eau à 15 mg/kg. De la manière indiquée sur la figure 1, la pression sanguine artérielle.moyenne a été réduite de 3680 Pa, 4,5 heures après l'administration.

## Revendications

1. Un composé **caractérisé en ce qu'**il est choisi parmi :
4,4'-dithiobis- (3-aminobutane-1- sulfonate de sodium) ;
4,4'-dithiobis-((2, 2-diméthylpropyle)3-aminobutane-1-sulfonate),
ainsi que leurs sels et produits de solvatation pharmaceutiquement acceptables.

2. Composé selon la revendication 1, **caractérisé en ce qu'**il est choisi parmi:
- le bis-chlorhydrate de 4,4'-dithiobis- (3-aminobutane-1-sulfonate de sodium) ;
- le bis-trifluoroacétate de 4,4'-dithiobis-((2, 2-diméthylpropyle)3-aminobutane-1-sulfonate).

3. Composé selon l'une des revendications 1 ou 2, **caractérisé en ce qu'**il est destiné à être utilisé en thérapeutique.

4. Composition pharmaceutique, **caractérisée en ce qu'**elle comprend un composé selon l'une des revendications 1 ou 2.

5. Utilisation d'un composé selon l'une des revendications 1 ou 2, pour la production d'un médicament pour le traitement de l'hypertension artérielle.

6. Utilisation d'un composé selon l'une des revendications 1 ou 2, pour la production d'un médicament pour le traitement des maladies du coeur, du système vasculaire périphérique ou cérébral, du cerveau, de l'oeil ou du rein, lesdites maladies étant directement ou indirectement liées à l'hypertension artérielle.

7. Utilisation selon l'une des revendications 5 ou 6 pour la production d'un médicament pour le traitement de maladies choisies parmi l'hypertension artérielle primaire et secondaire, un ichtus, l'ischémie myocardique, l'insuffisance cardiaque, l'insuffisance rénale, l'infarctus du myocarde, la protéinurie diabétique, le syndrome X et le glaucome.

## Claims

1. A compound, **characterized in that** it is selected from :
- 4,4'-dithiobis(sodium 3-aminobutane -1-sulfonic acid)
- 4,4'-dithiobis(2,2dimethylpropyl)-3-aminobutane-1-sulfonate
and the pharmaceutically acceptable salts and solvates thereof.

2. Compound according to claim 1, **characterized in that** it is selected from :
- 4,4'-dithiobis(sodium 3-aminobutane -1-sulfonate) bis chlorhydrate;
- 4,4'-dithiobis(2,2dimethylpropyl)-3-aminobutane-1-sulfonate) bis trifluoroacetate.

3. Compound according to any one of claims 1 or 2, **characterized in that** it is intended for use in therapy.

4. Pharmaceutical composition, **characterized in that** it comprises a compound according to any one of claims 1 or 2.

5. Use of a compound according to any one of claims 1 or 2, for the production of a medicament for treating arterial hypertension.

6. Use of a compound according to any one of claims 1 or 2, for the production of a medicament for treating heart diseases, diseases of the peripheral or cerebral vascular system, brain diseases, eye diseases or kidney diseases, said diseases being directly or indirectly linked to arterial hypertension.

7. Use according to any one of claims 5 or 6 for the production of a medicament for treating diseases selected from primary and secondary arterial hypertension, ictus, myocardial ischaemia, cardiac insufficiency, renal insufficiency, myocardial infarction, diabetic proteinuria, syndrome X and glaucoma.

## Patentansprüche

1. Verbindung, **dadurch gekennzeichnet, dass** diese ausgewählt ist aus der Gruppe bestehend aus 4,4'-Dithiobis-(3-aminobutan-1-natriumsulfonat), 4,4'-Dithiobis-((2,2-dimethylpropyl)3-aminobutan-1-sulfonat) und auch deren pharmazeutisch akzeptablen Salze und Solvate.

2. Verbindung nach Anspruch 1, **dadurch gekennzeichnet, dass** diese ausgewählt ist aus:
- dem Bis-Chlorhydrat von 4,4'-Dithiobis-(3-aminobutan-1-natriumsulfonat),
- dem Bis-Trifluoracetat von 4,4'-Dithiobis-((2,2-dimethylpropyl)3-aminobutan-1-sulfonat).

3. Verbindung nach einem der Ansprüche 1 oder 2, **dadurch gekennzeichnet, dass** diese für eine therapeutische Verwendung bestimmt ist.

4. Pharmazeutische Zusammensetzung, **dadurch gekennzeichnet, dass** diese eine Verbindung gemäß einem der Ansprüche 1 oder 2 umfasst.

5. Verwendung einer Verbindung gemäß einem der Ansprüche 1 oder 2 zur Herstellung eines Medikaments für die Behandlung von arterieller Hypertonie.

6. Verwendung einer Verbindung gemäß einem der Ansprüche 1 oder 2 zur Herstellung eines Medikaments zur Behandlung von Krankheiten des Herzens, des peripheren oder zerebralen Gefäßsystems, des Gehirns, des Auges oder der Niere, wobei die Krankheiten direkt oder indirekt mit der arteriellen Hypertonie verbunden sind.

7. Verwendung nach einem der Ansprüche 5 oder 6 zur Herstellung eines Medikaments zur Behandlung von Krankheiten ausgewählt aus primärer oder sekundärer arterieller Hypertonie, einem Ictus, Myokardischämie, Herzinsuffizienz, Niereninsuffizienz, Myocardinfarkt, diabetischer Proteinurie, dem X-Syndrom und dem Glaukom.
